# EUROPEAN PATENT APPLICATION

(11) **EP 4 751 763 A1**
(43) Date of publication of application: **03.06.2026**
(21) Application number: 24215833.5
(22) Date of filing: 27.11.2024
(51) Int. Cl.: A61N 1/04

(54) **HYDROGEL CONDUCTORS FOR STORAGE OF MEDICAL ELECTRODE PADS**

(71) Applicant: Koninklijke Philips N.V., 5656 AE Eindhoven (NL)
(72) Inventor: GREWELL, Bruce Kelly, 5656 AG Eindhoven (NL)
(74) Representative: Philips Intellectual Property & Standards

(57) **Abstract**

An electrode pad storage assembly (60) employing a pair of electrode pads (10) electrically connectable or electrically connected to an electrical treatment device (40) (e.g., an automatic external defibrillator), and an electrode pad storage device for facilitating a storage of the pair of electrode pads (10). Each electrode pad (10) includes a hydrogel layer (11). The electrode pad storage device includes a hardliner (20) whereby the pair of electrode pads (10) are releasably sealable or releasably sealed to the hardliner (20), and a hydrogel conductor (30) adjoined to the hardliner (20) and configured to establish electrical communication between the hydrogel layers (11) of the electrode pads (10) when the electrode pads (10) are releasably sealed to the hardliner (20).

## Description

### FIELD OF THE INVENTION

The present disclosure generally relates to a storage of a pair of medical electrode pads. The present disclosure particularly relates to establishing an electrical communication between a pair of medical electrode pads during a storage of the pads.

### BACKGROUND OF THE INVENTION

An automatic external defibrillator (AED) is easy to use and provides quick electrical therapy treatment for a sudden cardiac arrest patient as needed. More particularly, an AED can be designed to guide a responder through the rescue process, including cardiopulmonary resuscitation (CPR) and electrical current initiation, if needed. Non-limiting examples of such an AED include the HeartStart OnSite AED, the HeartStart FRx AED HeartStart Home AED.

Electrode pads can be electrically connectable or connected to the AED and attached to the sudden cardiac arrest patient for delivering the electrical therapy treatment to the sudden cardiac arrest patient as needed. When the AED is not in use, the electrode pads need to be stored in a manner that maintains reliable usage of the electrode pads during an electrical therapy treatment and enables accurate self-testing of the storage electrode pads. As such, the defibrillator industry is always striving to enhance electrode pad storage devices.

### SUMMARY OF THE INVENTION

The present disclosure describes an electrical conductor incorporating hydrogel material that establishes electrical communication between a pair of electrode pads, particularly self-storing electrode pads. The hydrogel conductor facilitates an AED or any other electrical treatment device electrically connected to the electrode pads to perform self-test and other functions of the electrode pads when the electrode pads are sealed to a hardliner during the shelf life of the AED or other electrical treatment device without any signification degradation to the electrode pads.

For purposes of describing and claiming the present disclosure, terms of the art of the present disclosure, but not limited to, "electrode pad", "electrical therapy", "hydrogel", "hardliner" and "release liner" are to be interpreted as known in the art of the present disclosure and as exemplary described in the present disclosure.

Furthermore, for purposes of describing and claiming the present disclosure, the term "hydrogel conductor" broadly encompasses an electrical conductive hydrogel material as known in the art of the present disclosure or hereinafter conceived.

The present disclosure may be embodied as (1) an electrode pad storage device, (2) an electrode pad storage assembly, and (3) an electrical treatment system.

Various embodiments of an electrode pad storage device of the present disclosure encompass a hardliner and a hydrogel conductor adjoined to the hardliner for facilitating a storage of a pair of electrode pads, each electrode pad including a hydrogel layer. For storage, the pair of electrode pads are releasably sealable or releasably sealed to the hardliner, and the hydrogel conductor establishes an electrical communication between the hydrogel layers of the electrode pads when the electrode pads are releasably sealed to the hardliner.

Various embodiments of an electrode pad storage assembly of the present disclosure encompass a pair of electrode pads, each electrode pad including a hydrogel layer, and further encompasses an electrode pad storage device of the present disclosure. For storage, the pair of electrode pads are releasably sealable or releasably sealed to the hardliner, and the hydrogel conductor establishes an electrical communication between the hydrogel layers of the electrode pad when the electrode pads are releasably sealed to the hardliner.

Various embodiments of an electrode pad storage system of the present disclosure encompass an electrical treatment device operable for providing electrical monitoring or an electrical therapy to a patient (e.g., an automatic external defibrillator or a semi-automatic external defibrillator), and further encompasses an electrode pad storage assembly of the present disclosure. For storage, the pair of electrode pads are releasably sealable or releasably sealed to the hardliner, and the hydrogel conductor establishes an electrical communication between the hydrogel layers of the electrode pad when the electrode pads are releasably sealed to the hardliner.

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1A illustrates an exemplary embodiment of an unassembled electrical therapy pad assembly in accordance with the present disclosure;
FIG. 1B illustrates an exemplary embodiment of an assembled electrical therapy assembly in accordance with the present disclosure;
FIGS. 2A and 2B illustrate a first exemplary embodiment of an unassembled electrode storage assembly in accordance with the present disclosure;
FIGS. 3A and 3B illustrate a second exemplary embodiment of an unassembled electrode storage assembly in accordance with the present disclosure;
FIG. 4A illustrates a first exemplary embodiment of a hydrogel conductor in accordance with the present disclosure;
FIGS. 4B and 4C illustrate a first exemplary embodiment of an electrode pad storage device in accordance with the present disclosure;
FIG. 5A illustrates a second exemplary embodiment of a hydrogel conductor in accordance with the present disclosure;
FIGS. 5B and 5C illustrate a second exemplary embodiment of an electrode pad storage device in accordance with the present disclosure;
FIG. 6A illustrates a third exemplary embodiment of a hydrogel conductor in accordance with the present disclosure; and
FIGS. 6B and 6C illustrate a third exemplary embodiment of an electrode pad storage device in accordance with the present disclosure.

### DETAILED DESCRIPTION OF THE PREFERRED EMBODIMENTS

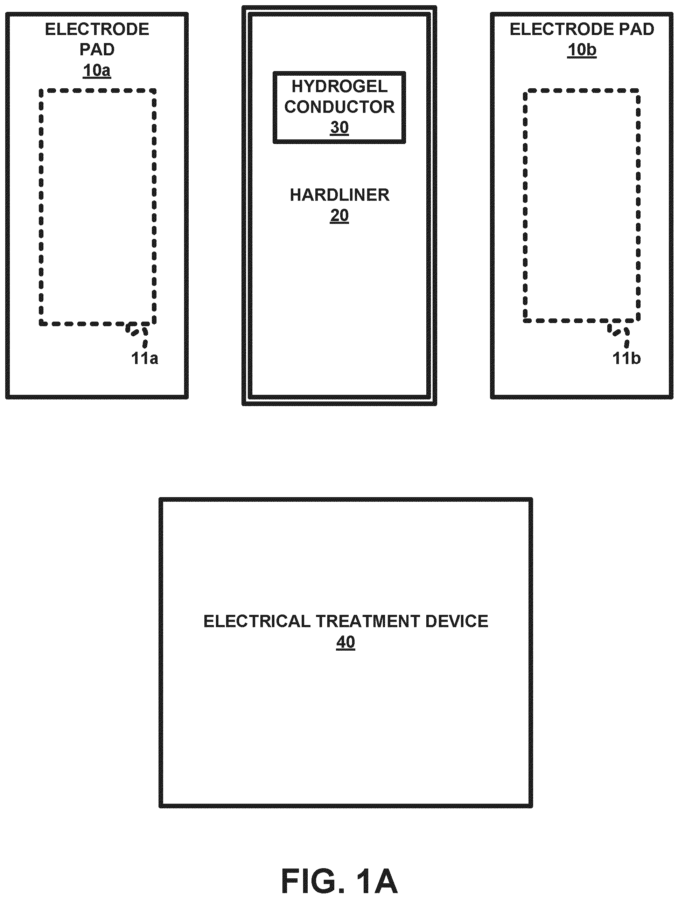

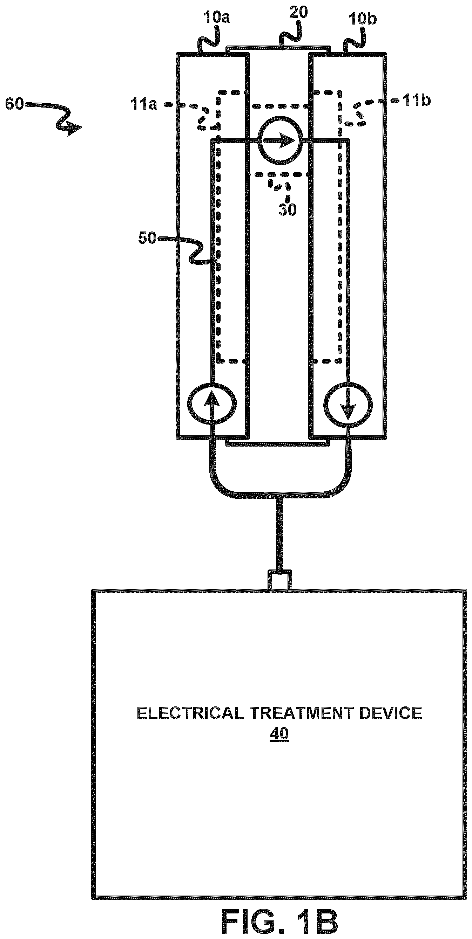

To facilitate an understanding of the present disclosure, the following description of FIGS. 1-6C describes and teaches exemplary embodiments of devices and assemblies in accordance with the present disclosure. From the description of FIGS. 1-6C, those having ordinary skill in the art of the present disclosure will appreciate how to apply the present disclosure to make and use additional embodiments of devices and assemblies of the present disclosure.

FIGS. 1A and 1B respectively illustrate an unassembled and an assembled electrode therapy assembly 60 of the present disclosure employing a pair of electrode pads 10a and 10b including electrically conductive hydrogel layers 11a and 11b as known in the art of the present disclosure and hereinafter conceived. Non-limiting examples include Smart Pads II and Smart Pads III as known in the art of the present disclosure.

Still referring to FIGS. 1A and 1B, the electrode therapy assembly 60 further employs a electrode pad storage device of the present disclosure including a hardliner 20 as known in the art of the present disclosure or hereinafter conceived, and a hydrogel conductor 30 of the present disclosure.

Hydrogel conductor 30 incorporates electrically conductive hydrogel material as known in the art of the present disclosure or hereinafter conceived, and can incorporate additional material for supplementing/complementing the hydrogel material as would be appreciated by those having ordinary skill in the art of the present disclosure.

Still referring to FIGS. 1A and 1B, the electrode therapy assembly further employs an electrical treatment device 40 for providing an electrical therapy to a patient as known in the art of the present disclosure or hereinafter conceived. Non-limiting examples of electrical treatment device 40 are an automated external defibrillator, a semi-automatic external defibrillator and a manual external defibrillator.

Still referring to FIGS. 1A and 1B, in practice, as would be understood by those having ordinary skill in the art of the present disclosure, electrode pads 10a and 10 are releasably sealable to hardliner 20 and electrically connectable to electrical treatment device 40 as shown in FIG. 1A, or electrode pads 10a and 10 are releasably sealed to hardliner 20 and electrically connected to electrical treatment device 40 as shown in FIG. 1B. When electrode pads 10a and 10b are releasably sealed to hardliner 20 and electrically connected to electrical treatment device 40 as shown in FIG. 1B, hydrogel conductor 30 establishes electrical communication between hydrogel layers 11a and 11b to thereby allow electrical treatment device 40 to perform self-test and other storage functions of the electrode pads 11a and 11b via an electrical path 50.

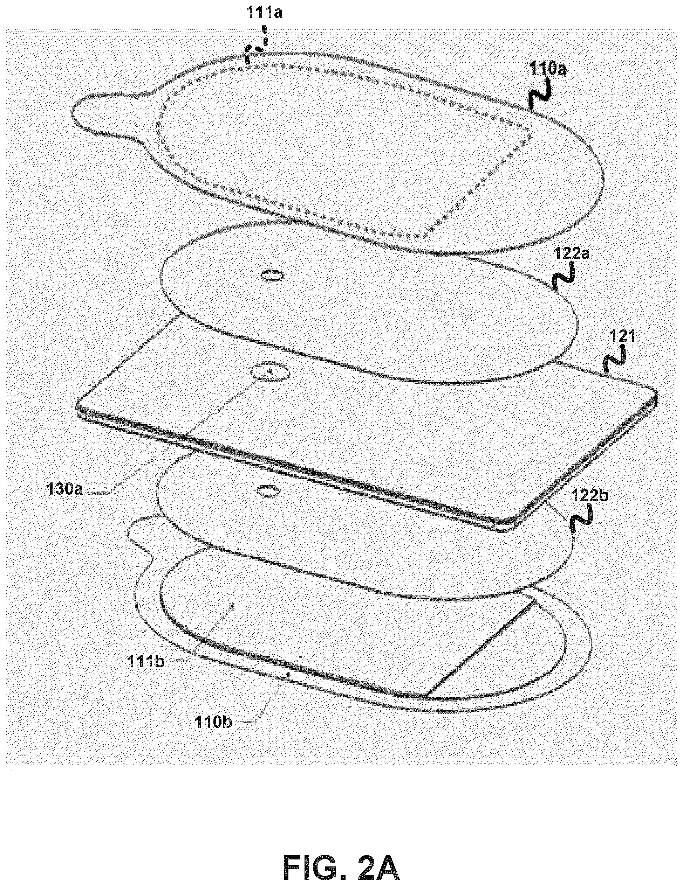

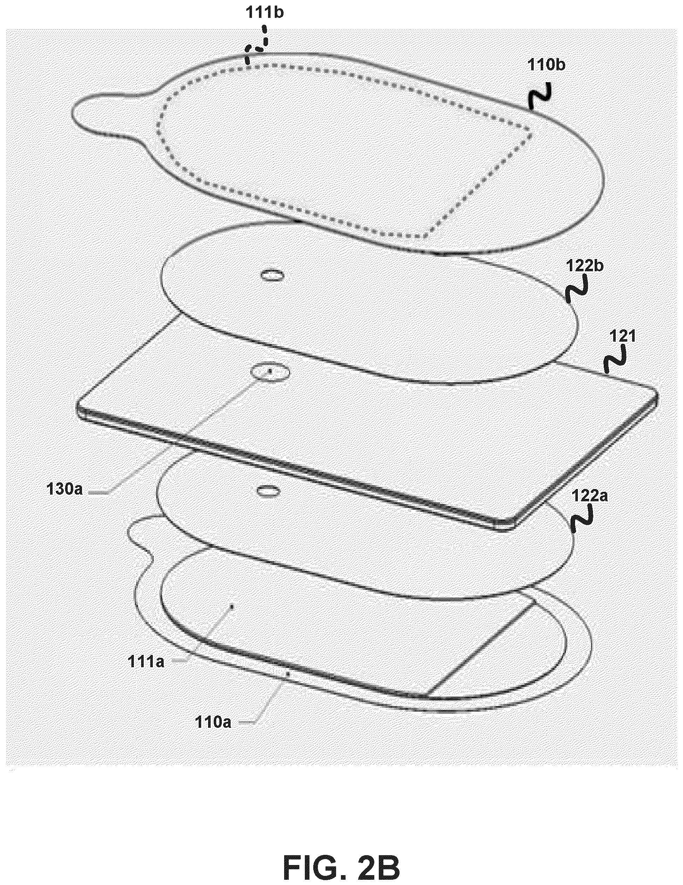

FIGS. 2A and 2B illustrate respective views of an unassembled electrode pad assembly of the present disclosure employing an electrode pad having a backing layer 110a and a hydrogel layer 111a, an electrode pad having a backing layer 110b and a hydrogel layer 111b, a hardliner 121a and a pair of release liners 122a and 122b, all as known in the art of the present disclosure or hereinafter conceived. Backing layer 110a and hydrogel layer 111a constitute an electrode, and backing layer 110b and hydrogel layer 111b constitute an electrode.

In practice, as would be appreciated by those having ordinary skill in the art of the present disclosure, the dimensions, shapes and material composition of backing layers 110a and 110b, hydrogel layers 111a and 111b, hardliner 121a and release liners 122a and 122b can vary.

This electrode pad storage assembly of the present disclosure further employs a hydrogel conductor 130a of the present disclosure. For this embodiment, the hydrogel material of hydrogel conductor 130a is poured and cured within a conduit of hardliner 121a and dimensioned to be flush with conduits of release liners 122a and 122b to thereby electrically connect with hydrogel layers 111a and 111b when the electrode pads are releasably sealed with hardliner 121a via backing layers 110a and 110b as known in the art of the present disclosure.

The hydrogel material can be poured and cured in place either before or after the release liners 122a and 122b are adhered to hardliner 121a.

Also in practice, hardliner 121a can include a structural support formed within the conduit of hardliner 121a. Non-limiting examples of such structural support include a mesh and an nonconductive obstructor as will be further described in the present application.

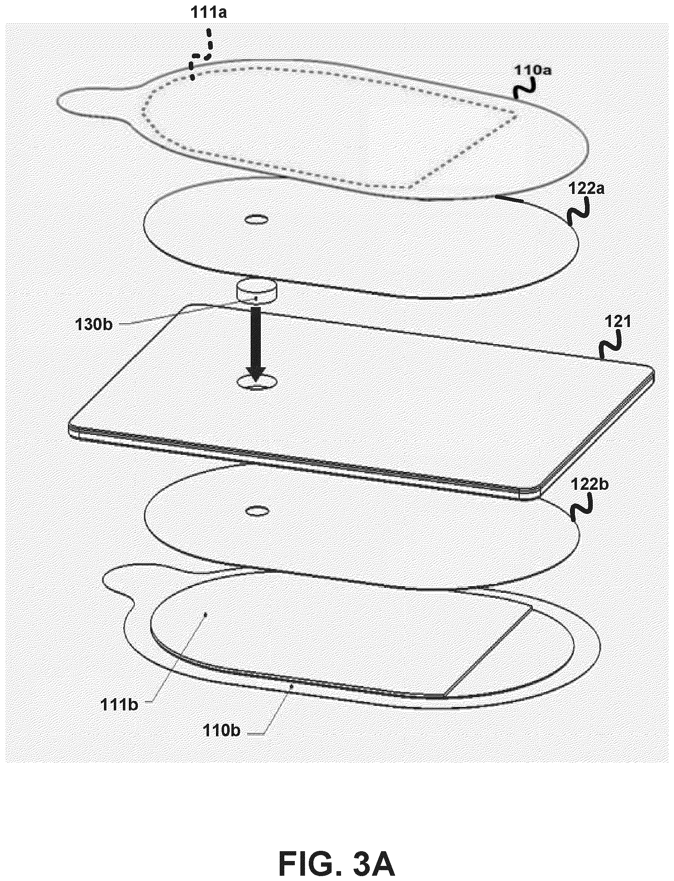

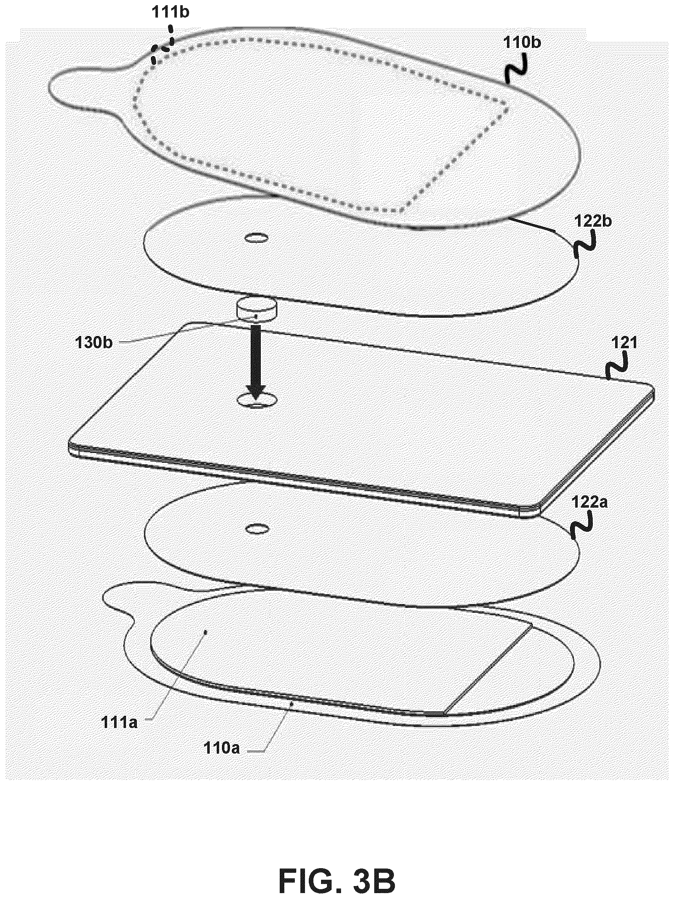

FIGS. 3A and 3B illustrate respective views of an additional exemplary embodiment of an unassembled electrode pad storage assembly of the present disclosure employing the electrode pad having a backing layer 110a and a hydrogel layer 111a, the electrode pad having a backing layer 110b and a hydrogel layer 11 1b, a hardliner 121b and the pair of release liners 122a and 122b, all as known in the art of the present disclosure or hereinafter conceived.

In practice, as would be appreciated by those having ordinary skill in the art of the present disclosure, the dimensions, shapes and material composition of backing layers 110a and 110b, hydrogel layers 111a and 111b, hardliner 121b and release liners 122a and 122b can vary.

This electrode pad storage assembly of the present disclosure further employs a hydrogel conductor 130b of the present disclosure. For this embodiment, the hydrogel material of hydrogel conductor 130b can be poured and cured in bulk a sheet form, then various conductive element shapes can be died cut whereby the hydrogel material can be arranged within a conduit of hardliner 121b and flush with conduits of release liners 122a and 122b to thereby electrically connect with hydrogel layers 111a and 111b when the electrode pads are releasably sealed with hardliner 121b via release liners 122a and 122b as known in the art of the present disclosure. The hydrogel material may be supported by a thin permeable membrane (scrim) during the die cutting process and thereafter.

In practice, hydrogel conductor 103b can include a structural support that is arrangeable within the conduit of hardliner 121a. Non-limiting examples of such structural support include a mesh and a nonconductive obstructor as will be further described in the present application.

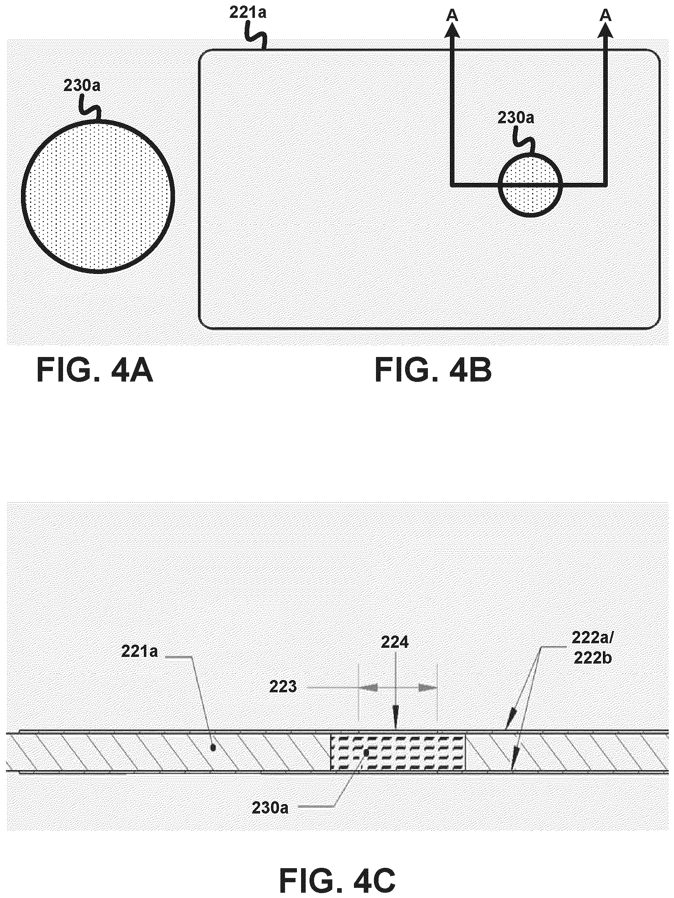

FIGS. 4A and 4B illustrate an exemplary embodiment of a hydrogel conductor 230a arranged within a conduit of a hardliner 221a. FIG. 4C illustrates a cross-section of hardliner 221a via line A-A of FIG. 4B.

Referring to FIG. 4C, during assembly of the exemplary embodiment of the electrode pad storage device of the present disclosure, a conduit of a lower release liner 222b is blocked while liquid hydrogel is poured through a conduit of an upper release liner 222a to fill in a conduit of liner 221a. The poured hydrogel is cured either before or after the transparent release liners 222a and 222b are adhered to the hardliner. The hydrogel is thickened to a high viscosity when cured and will not flow out of the conduit of hardliner 221a which is captured by the release liners 222a and 222b.

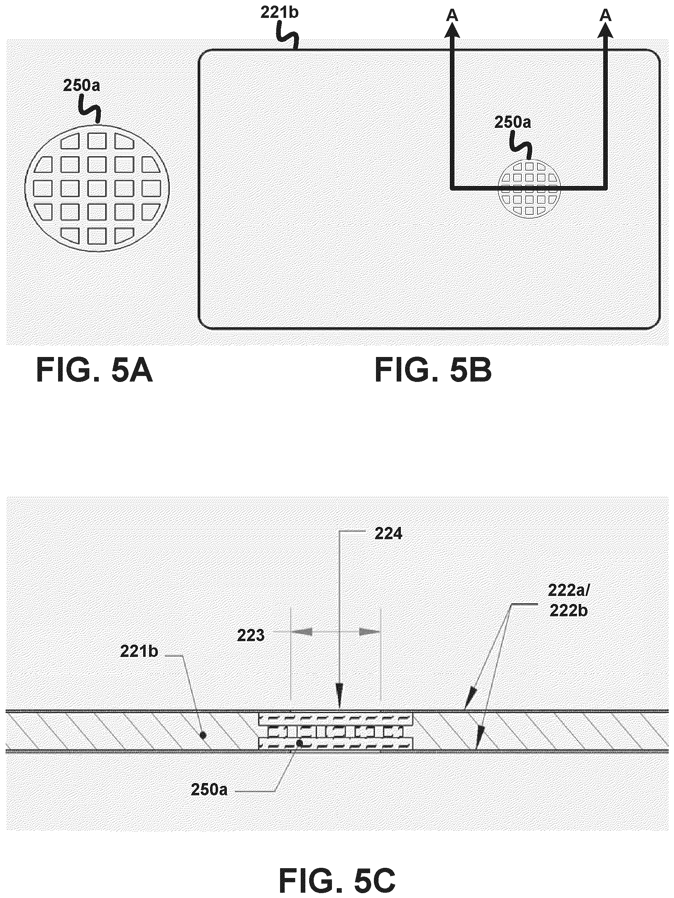

FIGS. 5A and 5B illustrate an exemplary embodiment of a mesh 250a molded/formed within a conduit of a hardliner 221b to facilitate the hydrogel pour and cure process by adding structural support for the cured hydrogel. FIG. 5C illustrates a cross-section of hardliner 221b via line A-A of FIG. 5B.

Referring to FIG. 5C, during assembly of the exemplary embodiment of the electrode pad storage device of the present disclosure, the hydrogel material can be poured around mesh 250a and cured in place either before or after the release liners 222a and 222b are adhered to the hardliner 221b.

Alternatively, the hydrogel material can be poured and cured around mesh 250a and thereafter mesh 250a can be arranged within the conduit of hardliner 221b.

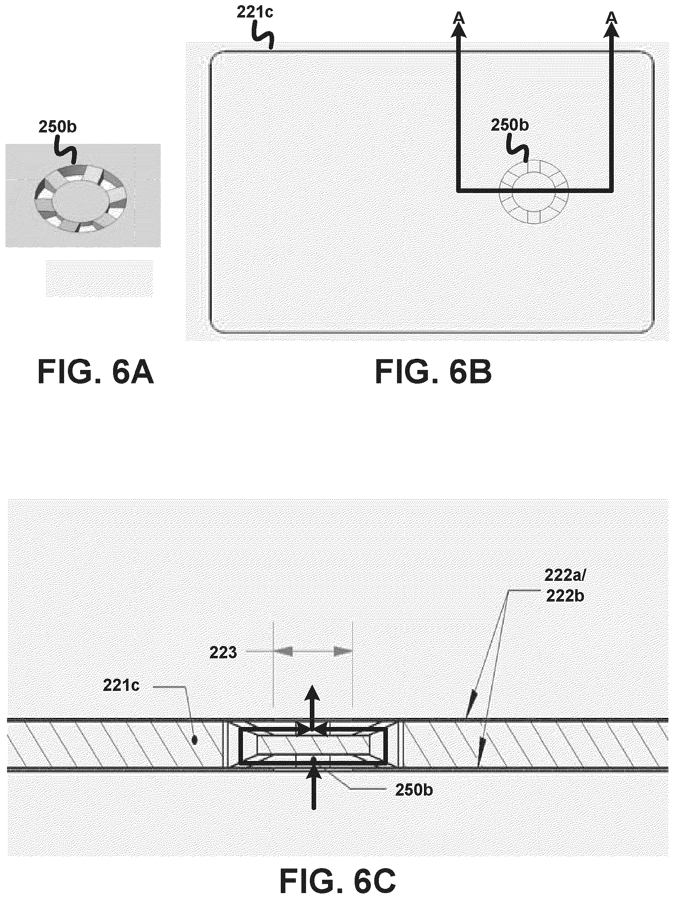

FIGS. 6A and 6B illustrate an exemplary embodiment of a nonconductive obstructor 250b molded/formed within a conduit of a hardliner 221c to facilitate the hydrogel pour and cure process by adding structural support for the cured hydrogel. Nonconductive obstructor 250b is designed to introduce an obstruction in the path of electrical current, which increases the distance the electrical current must travel for electrical continuity between the two opposing electrode pads when releasably sealed to hardliner 221c to thereby increase the electrical impedance between the two electrode pads.

FIG. 6C illustrates a cross-section of hardliner 221c via line A-A of FIG. 6B. Referring to FIG. 6C, during assembly of the exemplary embodiment of the electrode pad storage device of the present disclosure, the hydrogel material can be poured around nonconductive obstructor 250b and cured in place either before or after the release liners 222a and 222b are adhered to the hardliner 221c. The arrows symbolize the obstructed electrical path through nonconductive obstructor 250b.

Alternatively, the hydrogel material can be poured and cured around nonconductive obstructor 250b and thereafter nonconductive obstructor 250b can be arranged within the conduit of hardliner 221b.

From the description of FIGS. 1-6C herein, those having ordinary skill in the art will appreciate the numerous benefits of the present disclosure including, but not limited to, a hydrogel conductor of the present disclosure facilitating an AED or any other electrical treatment device electrically connected to the electrode pads to perform self-test and other functions of the electrode pads while the electrode pads are sealed to a hardliner during the shelf life of the AED or other electrical treatment device without any signification degradation to the electrode pads.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or an assembly in accordance with the present disclosure are also contemplated and considered to be within the scope of the present disclosure.

## Claims

1. An electrode pad storage device for facilitating a storage of a pair of electrode pads (10), each electrode pad (10) including a hydrogel layer (11), the electrode pad storage device comprising:
a hardliner (20) configured wherein the pair of electrode pads (10) are releasably sealable or releasably sealed to the hardliner (20); and
a hydrogel conductor (30) adjoined to the hardliner (20) and configured to establish electrical communication between the hydrogel layers (11) of the electrode pads (10) when the electrode pads (10) are releasably sealed to the hardliner (20).

2. The electrode pad storage device of claim 1,
wherein the hardliner (20) includes a conduit and a mesh formed within the conduit; and
wherein the hydrogel conductor (30) includes a hydrogel disposed within the mesh.

3. The electrode pad storage device of claim 1,
wherein the hydrogel conductor (30) includes a hydrogel disposed within a mesh; and
wherein the hardliner (20) includes a conduit and the mesh is arranged within the conduit.

4. The electrode pad storage device of claim 1,
wherein the hardliner (20) includes a conduit and a nonconductive obstructor formed within the conduit; and
wherein the hydrogel conductor (30) includes a hydrogel contiguous with the nonconductive obstructor.

5. The electrode pad storage device of claim 1,
wherein the hydrogel conductor (30) includes a hydrogel contiguous with a nonconductive obstructor; and
wherein the hardliner (20) includes a conduit and the nonconductive obstructor is arranged within the conduit.

6. An electrode pad storage assembly (60), comprising:
a pair of electrode pads, each electrode pad (10) including a hydrogel layer (11); and
an electrode pad storage device for facilitating a storage of the pair of electrode pads, the electrode pad storage assembly (60) including:
a hardliner (20) configured wherein the pair of electrode pads (10) are releasably sealable or releasably sealed to the hardliner (20); and
a hydrogel conductor (30) adjoined to the hardliner (20) and configured to establish electrical communication between the hydrogel layers (11) of the electrode pads (10) when the electrode pads (10) are releasably sealed to the hardliner (20).

7. The electrode pad storage assembly (60) of claim 6,
wherein the hardliner (20) includes a conduit and a mesh formed within the conduit; and
wherein the hydrogel conductor (30) includes a hydrogel disposed within the mesh.

8. The electrode pad storage assembly (60) of claim 6,
wherein the hydrogel conductor (30) includes a hydrogel disposed within a mesh; and
wherein the hardliner (20) includes a conduit and the mesh is arranged within the conduit.

9. The electrode pad storage assembly (60) of claim 6,
wherein the hardliner (20) includes a conduit and a nonconductive obstructor formed within the conduit; and
wherein the hydrogel conductor (30) includes a hydrogel contiguous with the nonconductive obstructor.

10. The electrode pad storage assembly (60) of claim 6,
wherein the hydrogel conductor (30) includes a hydrogel contiguous with a nonconductive obstructor; and
wherein the hardliner (20) includes a conduit and the nonconductive obstructor is arranged within the conduit.

11. An electrical therapy assembly (60), comprising:
an electrical treatment device (40) operable for providing an electrical monitoring or an electrical therapy to a patient; and
an electrode pad storage assembly (60) including:
a pair of electrode pads (10) electrically connectable or electrically connected to the electrical treatment device (40), each electrode pad (10) including a hydrogel layer (11);
an electrode pad storage device for facilitating a storage of the pair of electrode pad, the electrode pad storage assembly (60) including:
a hardliner (20) configured wherein the pair of electrode pads (10) are releasably sealable or releasably sealed to the hardliner (20); and
a hydrogel conductor (30) adjoined to the hardliner (20) and configured to establish electrical communication between the hydrogel layers (11) of the electrode pads (10) when the electrode pads (10) are releasably sealed to the hardliner (20).

12. The electrical therapy assembly (60) of claim 11,
wherein the hardliner (20) includes a conduit and a mesh formed within the conduit; and
wherein the hydrogel conductor (30) includes a hydrogel disposed within the mesh.

13. The electrical therapy assembly (60) of claim 11,
wherein the hydrogel conductor (30) includes hydrogel disposed within a mesh; and
wherein the hardliner (20) includes a conduit and the mesh is arranged within the conduit.

14. The electrical therapy assembly (60) of claim 11,
wherein the hardliner (20) includes a conduit and a nonconductive obstructor formed within the conduit; and
wherein the hydrogel conductor (30) includes hydrogel contiguous with the nonconductive obstructor.

15. The electrical therapy assembly (60) of claim 11,
wherein the hydrogel conductor (30) includes hydrogel contiguous with a nonconductive obstructor; and
wherein the hardliner (20) includes a conduit and the nonconductive obstructor is arranged within the conduit.
